# EUROPEAN PATENT APPLICATION

(11) **EP 3 293 800 A1**
(43) Date of publication of application: **14.03.2018**
(21) Application number: 16306117.9
(22) Date of filing: 07.09.2016
(51) Int. Cl.: H01M 2/34, H02J 7/00, A61M 5/142

(54) **MEDICAL DEVICE ALLOWING FOR A BATTERY DISCONNECTION**

(71) Applicant: Fresenius Vial SAS, 38590 Brézins (FR)
(72) Inventor: BERTHIER, Jean-Baptiste, 38210 Tullins-Fures (FR); FERRIGNO, Fabrice, 38000 Grenoble (FR)
(74) Representative: Kusche, Robert

(57) **Abstract**

A medical device (1) comprises an electronics unit (14) operative to control operation of the medical device (1), a battery device (17) operatively connected to the electronics unit (14) during operation of the medical device (1), a power supply connection device (19) for connecting the medical device (1) to an external power supply (4), and an actuation element (101) to switch the medical device (1) between an ON state in which the medical device (1) is ready-to-operate and an OFF state in which the medical device (1) is not ready-to-operate. The medical device (1) furthermore comprises a switching unit (18) operative to electrically disconnect the battery device (17) from the electronics unit (14) if the medical device (1) is in the OFF state and the power supply connection device (19) is disconnected from the external power supply (4). In this way, a medical device is provided which allows in an easy and convenient way to avoid a discharging of a battery device when the medical device is not in use.

## Description

The invention relates to a medical device according to the preamble of claim 1 and to a method for operating a medical device.

A medical device of this kind comprises an electronics unit operative to control operation of the medical device, a battery device operatively connected to the electronics unit during operation of the medical device, a power supply connection device for connecting the medical device to an external power supply, and an actuation element to switch the medical device between an ON state in which the medical device is ready-to-operate and an OFF state in which the medical device is not ready-to-operate.

A medical device of this kind may in particular be a medical pump constituted to administer a medical fluid, such as a medication or a nutritional fluid for the enteral or parenteral feeding, to a patient. A medical pump may for example be constituted by a volumetric (peristaltic) infusion pump or by a syringe infusion pump.

A medical device of this kind may be, during operation, powered by its battery device or by an external power supply to which the medical device is connected by means of its power supply connection device, for example a power line. For this, the electronics unit of a medical device, for example an electronics board comprising a control unit and a storage device, is connected to the battery device such that power may be supplied from the battery device to the electronics unit during operation of the medical device, for example during an infusion procedure (if the medical device is constituted by a medical pump).

Medical devices of this kind are for example used in homecare or in a hospital environment. Herein, medical devices are generally not continuously used, but may be held in storage for substantial amounts of time, during which the medical devices are switched off and are disconnected from an external power supply.

Although a medical device may be switched off (i.e., the actuation element is actuated to switch the medical device into the OFF state), electric currents may continue to flow from the battery device towards the electronics unit, such that the battery device may be continuously discharged and may enter into a deep discharge state, which eventually may lead to degradation of the battery performance.

It has been common practice to for instance physically disconnect the battery by removing the battery from the medical device in order to avoid a continuous discharging of the battery. This however is cumbersome and may not be expected in homecare or from hospital staff, for example a nurse. Furthermore, when a medical device again shall enter into operation, a physical reconnection of the battery is necessary, which again is cumbersome.

It is an object of the instant invention to provide a medical device and a method for operating a medical device which allow in an easy and convenient way to avoid a discharging of a battery device when the medical device is not in use.

This object is achieved by means of a medical device comprising the features of claim 1.

Accordingly, the medical device comprises a switching unit operative to electrically disconnect the battery device from the electronics unit if the medical device is in the OFF state and the power supply connection device is disconnected from the external power supply.

Hence, a switching unit is provided which shall serve to electrically disconnect the battery device from the electronics unit in case the medical device is not in use. Once the battery device is disconnected from the electronics unit, electrical currents can no longer flow from the battery device towards the electronics unit, such that a further discharge of the battery device is at least reduced or, beneficially, limited to leakage currents internally in the battery device.

The switching unit is operative to electrically disconnect the battery device from the electronics unit if it is detected that the actuation element has been actuated to switch the medical device into the OFF state and, in addition, if it is detected that the power supply connection device is disappearing from the external power supply. Hence, if the medical device is switched off and if the external power supply is no longer present to supply an external power, it is assumed that the medical device is ready to be put in storage, such that the switching unit electrically disconnects the battery device from the electronics unit.

By means of the switching unit, which for example may be constituted by a mechanical switch for opening a physical, electrical connection in between the battery device and the electronics unit, the battery may hence be isolated from the electronics unit, even if the battery device is fully charged. This allows to prolong the period over which the medical device may be placed in storage without the battery device entering into a deep discharge state.

The switching unit is operated and controlled internally within the medical device. Because the switching unit is automatically controlled, no additional action by a user is necessary to disconnect the battery device from the electronics unit in case the medical device shall be put into storage, and no special attention must hence be paid by a user to the battery device. Because a removal of the battery device from the medical device is not necessary, the housing of the medical device does not need to be opened and hence the risk for degrading the water tightness and for damaging wire connections internally in the medical device is reduced.

In one embodiment, the switching unit is operative to electrically reconnect the battery device to the electronics unit only if the power supply connection device is (again) connected to the external power supply. Hence, if it is detected that again a connection to an external power supply is present, the switching unit may be activated to reconnect the battery device to the electronics unit, such that the medical device once more becomes operative.

The reconnection takes place without a specific user interaction, that is, without a user having to perform a specific action to reconnect the battery device to the electronics unit. The reconnection takes place fully automatically.

The switching unit may be operative to detect if the power supply connection device is connected to or disconnected from an external power supply. The switching unit may be realized by an intrinsic hardware unit. In this way it can be ensured that a software error of a control device of the electronics unit may not cause a disconnection of the battery device. In addition, providing the switching unit as an intrinsic unit having additional and independent functions from the electronics unit may be beneficial for safety reasons in order to avoid an unintentional disconnection of the battery.

The electronics unit may, in one embodiment, comprise a control device such as a microcontroller and a storage device, which together serve to control the operation of the medical device, for example a pumping operation in case the medical device is constituted by a medical pump.

In another aspect, the switching unit may be operative to disconnect the battery device from the electronics unit if a charging level of the battery device is below a predefined threshold, even if the medical device is in the ON state. Hence, the switching unit may be operative to detect a charging level of the battery device, and if it is found that the charging level of the battery device is below the predefined threshold (that is, the charging level of the battery device is low), a disconnection of the battery device from the electronics unit takes place such that no further discharging of the battery device via the electronics unit can take place. This serves to protect the battery device from an extensive discharging.

As said, the medical device may be constituted by a medical pump for administering a medical fluid to a patient. A medical pump of this kind may for example be a volumetric (peristaltic) infusion pump or a syringe infusion pump. A medical device of this kind may be portable for example within a hospital or at homecare such that it may easily be placed on the bed side of a patient and may be moved within the hospital environment from one location to another.

The object is also achieved by a method for operating a medical device. The method comprises:
- providing a medical device comprising an electronics unit operative to control operation of the medical device, a battery device operatively connected to the electronics unit during operation of the medical device, a power supply connection device for connecting the medical device to an external power supply, and an actuation element to switch the medical device between an ON state in which the medical device is ready-to-operate and an OFF state in which the medical device is not ready-to-operate, and
- electrically disconnecting, using a switching unit, the battery device from the electronics unit if the medical device is in the OFF state and the power supply connection device is disconnected from the external power supply.

The advantages and advantageous embodiments described above for the medical device equally apply also to the method, such that it shall be referred to the above.

The idea of the invention shall subsequently be described in more detail with reference to the embodiments shown in the figures. Herein:
- Fig. 1: shows a view of a medical device constituted as a syringe pump; and
- Fig. 2: a schematic drawing of a medical device having an electronics unit, a battery device and a switching unit to disconnect the battery device from the electronics unit.

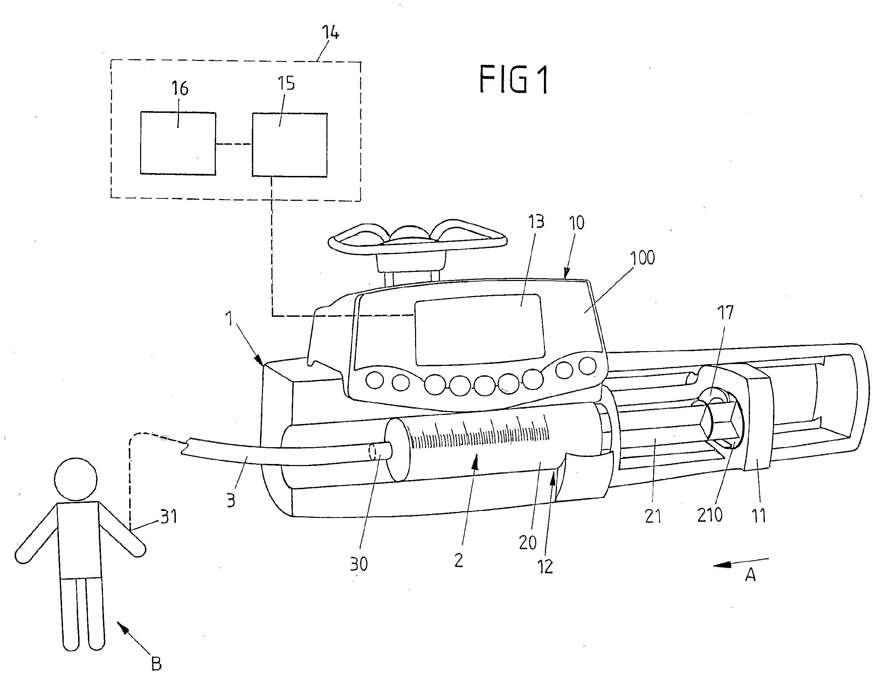

Fig. 1 shows, by way of example, an embodiment of a medical device 1 in the shape of a medical pump, in this case a syringe pump.

The medical device 1 comprises a housing 10 having a front face 100 and a display device 13 arranged thereon. The display device 13 may for example be a touch-sensitive display allowing a user to enter commands for operation of the medical device 1 and displaying operational information regarding the process of an actual infusion operation.

The medical device 1 comprises a receptacle 12 in which a syringe 2 having a cylindrical tube 20 is arranged. A piston 21 is movable within the cylindrical tube 20 and is in engagement with a pusher device 11 of a pumping mechanism of the medical device 1. At an end of the cylindrical tube 20 opposite the piston 21 a delivery line 3 extends from the cylindrical tube 20 towards a patient B, the delivery line 3 being connected to the cylindrical tube 20 at a first end 30 and to the patient B at a second end 31.

The piston 21 comprises a head 210 facing away from the cylindrical tube 20 and being in abutment with the pusher device 11 of the medical device 1. During operation of the medical device 1, the pusher device 11 is electromotorically driven in an actuation direction A such that the piston 21 is moved into the cylindrical tube 20 and a medical fluid contained in the cylindrical tube 20 is delivered via the delivery line 3 towards the patient B.

The medical device 1 comprises an electronics unit 14, for example an electronics board, including a processor device 15 and a storage device 16. Via the processor device 15 the infusion operation of the medical device 1 is controlled. In the storage device 16 operational parameters, such as mechanical characteristics of the syringe 2 used on the medical device 1 as well as operational data, may be stored.

During an infusion process a medical fluid, for example a medication or a nutritional fluid for the enteral or parenteral feeding of a patient or the like, is delivered from the cylindrical tube 20 via the delivery line 3 towards the patient B. For this, the piston 21 is continuously pushed into the cylindrical tube 20 in the actuation direction A such that a desired flow rate is obtained, which is programmed by a user prior to the start of the infusion operation.

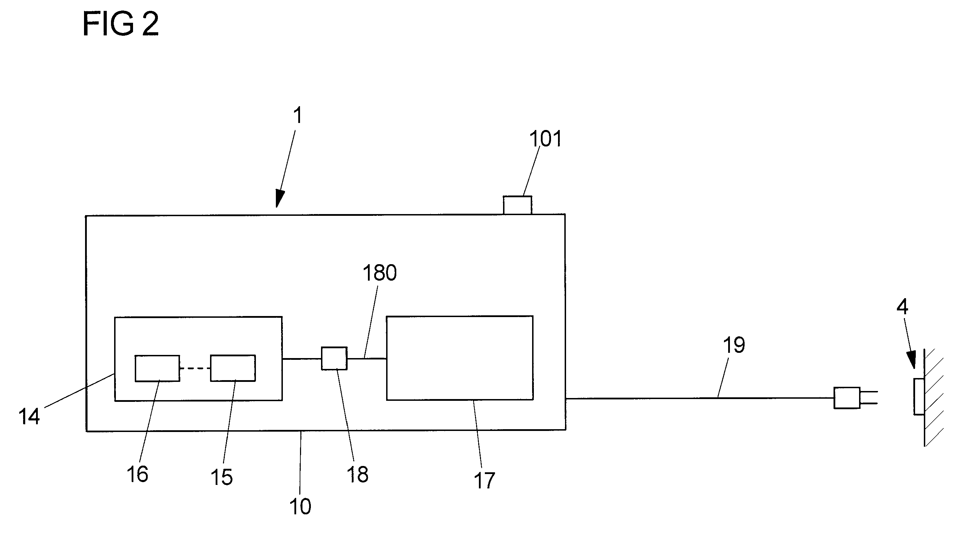

As schematically shown in Fig. 2, the medical device 1 comprises a battery device 17 connected to the electronics unit 14 via a connection 180 such that the electronics unit 14 with its control device 15 and the storage device 16 may be powered by the battery device 17. In addition, the medical device 1 comprises a power supply connection device 19 for example in the shape of a power line which may be connected, for example via a plug and socket connection, to an external power supply 4 such that, via the power supply connection device 19, the medical device 1 may also be powered by the external power supply 4.

During normal operation, the medical device 1 is powered by the battery 17 or by the external power supply 4. When the medical device 1 is connected to the external power supply 4, the battery device 17 herein is also charged.

The medical device 1 can be switched off by actuating an actuation element 101 in the shape of a button or the like such that the medical device 1, after operation, may be transferred into an OFF state in which substantial functions of the medical device 1 are switched off and are no longer operable.

In particular in a hospital or in homecare environment medical devices 1 herein may not be used continuously and may be placed in storage over longer periods of time during which they are not in use. Hence, during those storage periods, the medical devices 1 generally are switched off and are disconnected from an external power supply 4.

To avoid, during those storage periods, that the battery device 17 may be discharged by currents flowing over the connection 180 towards the electronics unit 14, a switching unit 18 is provided in the medical device 1 which serves to disconnect the battery device 17 from the electronics unit 14 in case it is detected that the medical device 1 is switched off and, in addition, is disconnected from an external power supply 4. The switching device 18 may for example be implemented by a mechanical switch which is constituted to physically and electrically open the connection 180 in between the battery device 17 and the electronics unit 14, such that no currents can flow in between the battery device 17 and the electronics unit 14.

The switching unit 18 may be implemented as a modular, intrinsic unit within the medical device 1 which is independent from the electronics unit 14. In particular, the switching unit 18 may be operative to detect whether the medical device 1 is in the ON state or in the OFF state and for this may for example detect a switching position of the actuation element 101. In addition, the switching unit 18 is operative to detect whether the medical device 1 is connected to an external power supply 4, for example by detecting a voltage level on the power supply connection device 19.

If a medical device 1 hence shall be placed in storage, and if the medical device 1 for this is switched off and is disconnected from an external power supply 4, the switching unit 18 disconnects the battery device 17 from the electronics unit 14, such that a discharge of the battery device 17 during storage is avoided or at least reduced. This may allow to prolong the storage period without the battery device 17 entering into a deep discharge state, which otherwise eventually may degrade the battery performance.

Because the switching unit 18 is implemented by an independent, intrinsic unit and is in particular separated from the electronics unit 14, it may be easily integrated into the medical device 1 and is furthermore not influenced by a potential software error of a software running on the control device 15. Hence, a faulty disconnection of the battery device 17 due to software malfunction cannot occur.

The switching unit 18 disconnects the battery device 17 from the electronics unit 14 automatically without requiring an additional user interaction, but by detecting the ON/OFF state of the medical device 1 and by detecting the disconnection of the power supply connection device 19 and by detecting there is no more electronic activity. Hence, no tempering of the housing 10 of the medical device 1 to physically remove the battery device 17 is required, hence avoiding a risk of degrading the water tightness of the housing 10 and of damaging wire connections within the medical device 1.

The switching unit 18 may, in addition, be constituted to disconnect the battery device 17 from the electronics unit 14 if it is found that the charging level of the battery device 17 falls below a predefined threshold. Hence, if it is found that the charging level of the battery device 17 is low, the switching unit 18 disconnects the battery device 17 from the electronics unit 14, such that a further discharge of the battery device 17 is avoided.

This disconnection of the battery device 17 due to a low charging level is independent of the disconnection when switching off the medical device 1 and when disconnecting the medical device 1 from an external power supply 4. The disconnection due to a low battery charging level may take place even if the medical device 1 is switched on (and the medical device 1 is not connected to an external power supply 4).

A workflow for disconnecting the battery device 17 from the electronics unit 14 is given in Table 1 below.

**TABLE 1**

| Step | ON/OFF button | Main power supply | Device status | Display | Battery / UC board |
|---|---|---|---|---|---|
| D1 | Released | Present | ON | Menu | Connected |
| D2 | Pressed | Present | Switching OFF | 3 ... 2 ... 1 | Connected |
| D3 | Pressed | Present | OFF | Battery pictogram | Connected |
| D4 | Pressed | Disappears | OFF | Blank, then battery pictogram | Connected |
| D5 | Pressed | Absent | Switching into sleep mode | Switching blank | Connected |
| D6 | Pressed | Absent | Sleep mode | Blank | Disconnecting |
| D7 | Pressed | Absent | Sleep mode | Blank | Disconnected |

In Table 1, the first column states different steps D1-D7, the second column relates to the state of the actuation element 101 for each step, the third column states the state of connection to an external power supply 4 (present or absent), the fourth column indicates the device state, the fifth column indicates what is shown on the display device 13, and the sixth column indicates whether the battery device 17 is connected to the electronics unit 14 or not.

Namely, in step D1 the actuation element 101 (ON/OFF button) is released such that the medical device is in its ON state, and the power supply connection device 19 is connected to an external power supply 4. The medical device 1 hence is in its ON state, and on the display 13 a regular menu is shown. In this state, the battery device 17 is connected to the electronics unit 14.

In step D2, the actuation element 101 is actuated to switch off the medical device 1, such that the pump status changes from ON to OFF and in step D3 is off. The display 13 now shows a battery pictogram, wherein the battery device 17 still is connected to the electronics unit 14.

In step D4 the connection of the power supply connection device 19 to the external power supply 4 is released, causing in step D5 the medical device 1 to switch into a sleep mode, in which the electronics unit 14 no longer performs any action. The display device 13 now shows a blank screen.

In step D6, now, the switching unit 18 disconnects the battery device 17 from the electronics unit 14, such that in step D7 the battery device 17 is electrically disconnected from the electronics unit 14. Hence, no discharging of the battery device 17 towards the electronics unit 14 may take place.

Pressing the ON/OFF button (actuation element 101) in the state of step D7 also allows to check the connection status of the battery: if the ON/OFF button is switched on in the state of step D7, the device 1 must not switch ON, due to the disconnection of the battery device 17.

A workflow for reconnecting the battery is stated in Table 2 below.

**TABLE 2**

| Step | ON/OFF button | Mains power supply | Device status | Display | Battery / UC board |
|---|---|---|---|---|---|
| R1 | - | Absent | Sleep mode | Blank | Disconnected |
| R2 | - | Present | Waking up | Battery pictogram | Re-connection |
| R3 | - | Present | ON | Menu | Connected |

Again, the first column states different steps R1-R3, the second column relates to the state of the actuation element 101 for each step, the third column states the state of connection to an external power supply 4 (present or absent), the fourth column indicates the device state, the fifth column indicates what is shown on the display device 13, and the sixth column indicates whether the battery device 17 is connected to the electronics unit 14 or not.

In step R1, the medical device 1 is not connected to an external power supply 4 and is in its sleep mode, the display device 13 showing a blank screen. In this state the battery device 17 is disconnected from the electronics unit 14. (Step R1 equals step D7 of Table 1 above.)

In step R2 the power supply connection device 19 is connected to an external power supply 4, causing the medical device 1 to wake-up, the display device 13 showing a battery pictogram. The switching unit 18 now reconnects the battery device 17 to the electronics unit 14.

In step R3 the medical device 1 is connected to the external power supply 4, and the medical device 1 is operative. The display device 13 shows a regular menu for operation, and the battery device 17 is connected to the electronics unit 14.

The idea underlying the invention is not limited to the embodiments described above, but may be implemented in an entirely different fashion in entirely different embodiments.

In particular, the invention is not limited to the medical devices in the shape of medical pumps, but may be employed also in other medical devices.

### List of Reference Numerals

- 1: Medical device
- 10: Housing
- 100: Front face
- 101: Actuation element (ON/OFF button)
- 11: Pusher device
- 12: Receptacle
- 13: Display device
- 14: Electronics unit (electronics board)
- 15: Processor device
- 16: Storage device
- 17: Battery device
- 18: Switching unit
- 180: Connection
- 19: Power supply connection device
- 2: Pumping device (syringe)
- 20: Cylindrical tube
- 21: Piston
- 3: Delivery line
- 30,31: End
- 4: External power supply
- A: Actuation direction
- B: Patient

## Claims

1. Medical device (1), comprising:
- an electronics unit (14) operative to control operation of the medical device (1),
- a battery device (17) operatively connected to the electronics unit (14) during operation of the medical device (1),
- a power supply connection device (19) for connecting the medical device (1) to an external power supply (4), and
- an actuation element (101) to switch the medical device (1) between an ON state in which the medical device (1) is ready-to-operate and an OFF state in which the medical device (1) is not ready-to-operate,
**characterized by**
a switching unit (18) operative to electrically disconnect the battery device (17) from the electronics unit (14) if the medical device (1) is in the OFF state and the power supply connection device (19) is disconnected from the external power supply (4).

2. Medical device (1) according to claim 1, **characterized in that** the switching unit (18) is operative to electrically re-connect the battery device (17) to the electronics unit (14) if the power supply connection device (19) is connected to the external power supply (4).

3. Medical device (1) according to claim 1 or 2, **characterized in that** the electronics unit (14) comprises a control device (15) and a storage device (16) for controlling operation of the medical device (1).

4. Medical device (1) according to one of claims 1 to 3, **characterized in that** the switching unit (18) is operative to disconnect the battery device (17) from the electronics unit (14) if a charging level of the battery device (17) is below a predefined threshold.

5. Medical device (1) according to one of the preceding claims, **characterized in that** the medical device (1) is a medical pump for administering a medical fluid, such as a nutritional fluid for the enteral or parenteral feeding, to a patient (B).

6. Method for operating a medical device (1), comprising:
- providing a medical device (1) comprising an electronics unit (14) operative to control operation of the medical device (1), a battery device (17) operatively connected to the electronics unit (14) during operation of the medical device (1), a power supply connection device (19) for connecting the medical device (1) to an external power supply (4), and an actuation element (101) to switch the medical device (1) between an ON state in which the medical device (1) is ready-to-operate and an OFF state in which the medical device (1) is not ready-to-operate,
**characterized by**
electrically disconnecting, using a switching unit (18), the battery device (17) from the electronics unit (14) if the medical device (1) is in the OFF state and the power supply connection device (19) is disconnected from the external power supply (4).
